# EUROPEAN PATENT SPECIFICATION

(11) **EP 0 851 914 B1**
(45) Date of publication and mention of the grant of the patent: **11.06.2003**
(21) Application number: 96935820.9
(22) Date of filing: 13.09.1996
(51) Int. Cl.: C12N 9/42, D21C 9/10

(54) **PURIFIED MANNANASE FROM BACILLUS AMYLOLIQUEFACIENS AND METHOD OF PREPARATION**
MANNASE VON BACILLUS AMYLOLIQUEFACIENS UND METHODE ZU IHRER PREPARATION
MANNANASE PURIFIEE PROVENANT DE BACILLUS AMYLOLIQUEFACIENS ET PROCEDE DE PREPARATION

(30) Priority: 20.09.1995 US 530801
(43) Date of publication of application: 08.07.1998
(73) Proprietor: GENENCOR INTERNATIONAL, INC., Palo Alto, California 94304 (US)
(72) Inventor: BODIE, Elizabeth A.,, Belmont , CA 94002 (US); CUEVAS, William A.,, San Francisco,CA 94114 (US); KANTELINEN, Anne K., 42300 Jämsänkoski (FI)
(74) Representative: Wibbelmann, Jobst, Dr., Dipl.-Chem.
(86) International application number: US9614692
(87) International publication number: WO97011164

(56) References cited:
- WO-A-91/18974
- WO-A-93/24622
- US-A- 5 296 223
- DATABASE WPI Section Ch, Week 9115 Derwent Publications Ltd., London, GB; Class D16, AN 91-105669 XP002022216 "New beta-mannanase hydrolyses beta 1,4-D-mannopyranoside bonds of mannan, glucomannan and can be manufactured at low cost." & JP,A,03 047 076 (AKINO T) , 28 February 1991 cited in the application
- DATABASE WPI Section Ch, Week 8816 Derwent Publications Ltd., London, GB; Class D16, AN 88-108330 XP002022217 "Production of beta-mannanase which hydrolyses beta-1,4-d-mannopyranoside bonds of e.g. glucomannan etc. and produces manno:oligo:saccharide(s)." & JP,A,63 056 289 (SHINGIJUTSU KAIHATSU, AKINO T) , 10 March 1988
- APPLIED AND ENVIRONMENTAL MICROBIOLOGY, vol. 56, no. 11, November 1990, pages 3505-3510, XP000615628 TALBOT G. ET AL.: "Purification and characterization of thermostable beta-mannanase and alpha-galactosidase from Bacillus stearothermophilus." cited in the application
- ENZYME MICROB. TECHNOL., vol. 14, 2 February 1992, pages 90-95, XP000615629 ROSS N.W. ET AL.: "Enzymic hydrolysis of water-soluble lignin-carbohydrate complexes formed from Populus deltoides: Effects of combinations of beta-mannanases, xylanase and acetyl xylan esterase."

## Description

### BACKGROUND OF THE INVENTION

The present invention relates to a novel mannanase enzyme. More specifically, the present invention is related to a novel mannanase obtained from *Bacillus amyloliquefaciens*, and production, purification and use of that mannanase, particularly in the bleaching of pulp.

Industrial uses of hemicellulases have become commercially important in recent years. In addition to animal feed and textiles, pulp and paper applications using hemicellulases continue to expand. While the paper industry has found use for enzymes in pitch control, de-watering and de-inking, these uses are generally still experimental. However, pulp pre-bleaching using xylanase is an established technology. It is believed that xylanase hydrolyzes reprecipitated xylans which are associated with chromophoric lignin on the surfaces of kraft pulp, resulting in improved lignin extractability and bleaching efficiency. With the use of xylanase, consumption of chlorine chemicals is reduced, which significantly reduces levels of hazardous waste released into mill waste streams, halogenated organic compounds being a major by-product of bleaching processes utilizing chlorine and chlorine-containing compounds. As environmental regulations concerning pulp mill effluents increase, chlorine-free alternatives for pulp bleaching will become critical for the paper manufacturing industry. In chlorine-free bleaching sequences, enzymes have been successfully used for increasing brightness or improving pulp quality, in part due to the decreased need for hydrogen peroxide.

Mannan and glucomannans are hemicelluloses associated with xylan and lignin on pulp surfaces and inner layers. As a result, pretreatment of pulp with a combination of mannanase and xylanase has been shown to result in higher brightness than that which can be obtained with xylanase alone (see e.g., Ross et al., Enzyme-Microb. Technol., vol. 14, no. 2, pp. 90-95 (1992)).

Mannanases have been identified in several *Bacillus* organisms. For example, Talbot et al., Appl. Environ. Microbiol., vol. 56, no. 11, pp. 3505-3510 (1990) describes a β-mannanase derived from *Bacillus stearothermophilus* in dimer form having a molecular weight of 162,000 daltons and an optimum pH of 5.5-7.5. Mendoza et. al., World J. Microbio. Biotech., vol. 10, no. 5, pp. 551-555 (1994) describes a β-mannanase derived from *Bacillus subtilis* having a molecular weight of 38,000 daltons, an optimum activity at pH 5.0 and 55°C and a pl of 4.8. JP 0304706, Derwent Accession No. 91-07734, describes a β-mannanase derived from *Bacillus sp*. having a molecular weight of 37,000 +/-3,000 daltons when measured by gel filtration, an optimum pH of 8-10 and a pl of 5.3-5.4. The prior art, however, fails to identify a mannanase derived from *Bacillus amyloliquefaciens*, as described below.

### SUMMARY OF THE INVENTION

According to the present invention, a purified mannanase enzyme obtainable from *Bacillus amyloliquefaciens* is provided. Preferably, the mannanase of the invention has a molecular weight of about 35,000 when analyzed with the SDS-PAGE method and 20,000 when measured by gel filtration, an isoelectric point (pl) of about 5.2-5.6, a pH optimum of about 4.8-5.2 and a half-life at 80°C of about 45 seconds. In another embodiment of the invention, a method is provided for the preparation of a purified mannanase derived from *Bacillus amyloliquefaciens*.

In another embodiment of the invention, the mannanase of the invention is used in the bleaching of pulp and paper. Preferably, the mannanase is used in combination with a xylanase.

### DETAILED DESCRIPTION OF THE INVENTION

According to a preferred embodiment of the invention, a fermentation broth of *Bacillus amyloliquefaciens* is prepared. After separation of whole cells, cell fragments and particulate matter, the supernatant is treated so as to concentrate and isolate the mannanase in the supernatant. In a preferred embodiment of the invention, the purified mannanase comprises a molecular weight of about 33-37 kD as determined with the SDS-PAGE method and about 18-22 kD as determined with the gel filtration method, a pl of about 5.2-5.6, a pH optimum of about 4.8-5.2 and a half life of about 45 seconds at 80 °C. Most preferably, the purified mannanase comprises a molecular weight of about 35 kD as determined with the SDS-PAGE method and about 18-22 kD as determined with the gel filtration method, a pl of about 5.4 and a pH optimum of about 5.0.

Fermentation of *Bacillus amyloliquefaciens* to produce mannanase according to the invention can be accomplished according to any art-recognized method of culturing this microorganism. Preferably, such conditions are manipulated to maximize mannanase production, which conditions are well known in the art.

Purification of the mannanase from the fermentation broth can be by any art-recognized means for purifying such composition. For example, filtration, centrifugation, chromatography, gel filtration or ultrafiltration are all suitable means of purifying the mannanase.

In another embodiment of the invention, the mannanase of the invention is used in the bleaching of pulp for the production of paper. While the use of mannanase alone in the bleaching of pulp provides an incremental benefit, it is particularly preferred to use mannanase of the invention in combination with xylanase to provide excellent bleaching results. The enzyme treatment (mannanase or mannanase/xylanase) can be utilized at any phase of the bleaching process, however, it is particularly preferred that the mannanase be used prior to bleaching with chlorine-containing chemicals. It is further preferred that the enzyme bleaching be used in combination with oxygen bleaching to limit or even eliminate the use of chlorine-containing chemicals from the bleaching process.

A suitable dosage for mannanase during pulp bleaching is about 10-500 nkat/g pulp when activity is measured by the DNS assay. Conditions for pulp treatment with mannanase are readily ascertained by those of skill in the art, however, a suitable temperature is about 40°C to about 60°C and a suitable pH is from about 4.0 to about 7.0. A suitable dosage for xylanase when used in combination with mannanase is about 0.10 to 200 units/g of dry pulp, and more preferably 0.50 to 50 units/g. The xylanase activity of the enzyme preparations is determined as follows: To 1.8 ml of xylan solution (0.6% Sigma No. X-0627, prepared in 0.05 m sodium acetate buffer and adjusted to pH 5.3 with acetic acid), 0.200 ml of suitably diluted enzyme in the same buffer is added. The solution is incubated at 40°C for exactly 30 minutes. The reaction is then stopped by adding 3 ml DNS reagent (3,5-dinitrosalicylate 10 g/l; Na,K tartrate 300 g/l), and the color is developed by boiling the sample for 5 minutes. The absorbency is then measured at a wave length of 540 nm. One enzyme unit liberates one micromole of reducing sugars calculated at xylose per minute under assay conditions. The activity is calculated from an enzyme dilution liberating 4 micromoles of reducing sugar under assay conditions.

The present invention may be applied to improve any of a wide variety of raw or processed pulps. Processed pulps, i.e., pulps which have been already previously treated to reduce their lignin content, are preferably treated in the process according to the invention to further enhance lignin removal. The present invention is preferably applied to treat softwood pulps to enhance lignin removal and brightening of the pulps. The invention is particularly applicable to chemical pulps, i.e., those in which the lignin component has been chemically modified by various chemical treatments such as in the sulfate (kraft) or sulfite processes and/or oxygen delignification, and is preferably applied to kraft pulps. In a preferred method, the enzymes of the present invention are applied to the pulp after kraft digestion or oxygen delignification but prior to chemical bleaching. In the case where both kraft digestion and oxygen delignification are performed on the same pulp, the enzyme is preferably applied after oxygen delignification. The present invention is also applicable to ozone bleached pulps or pulps which are bleached in ozone containing sequences.

Extractants are often used in pulp bleaching to remove the modified lignin component subsequent to bleaching. in a preferred embodiment of the invention, pulp treated with the enzymes of the present invention is subsequently treated with lignin-degrading chemicals such as chlorine, chlorine dioxide and peroxide, and then with an appropriate extractant. In yet another embodiment, the enzyme treated pulp may be treated with an appropriate extractant, followed by lignin degradation with chemical bleaching or enzymes and a subsequent final treatment with an appropriate extractant. Extractants which solubilize the affected lignin component include bases such as alkali metal hydroxides (E), DMF, dioxane, acetone, and alcohol. Hydroxide extractions may be combined with hydrogen peroxide (Eₚ) or oxygen (Eₒ). The resulting pulp may then be further bleached by a chemical bleaching sequence such as chlorine dioxide (DED) or peroxide (P-P) to the desired brightness. Substantial savings of chemicals are observed when the method of the present invention is practiced in comparison with preparing pulp bleached to the same brightness by the same sequence except without using the enzyme treatment by reducing the amount of chlorine-containing chemicals or peroxide used. Similarly, by performing the present invention with the above presented enzymes, one may apply the same amount of bleaching chemicals to the pulp and yet achieve a greater brightness in the treated pulp.

The invention will be further described by reference to the following examples, which examples are illustrative in purpose and are not intended to be limiting.

### EXAMPLES

### Example 1

### Purification of Mannanase from Fermentation Broth of Bacillus amyloliquefaciens

*Bacillus amyloliquefaciens*, ATCC #23842, was grown under conditions including 1% Proflo, 1% locus bean gum and 67.5 ml/l of a medium comprising 8.2 g/l KH₂PO₄, 91.2 g/l Na₂HPO₄.7H₂O, 5 g/l MgSO₄.7H₂O, 10.4 g/l KCl, 11.8 g/l sodium citrate.2H₂O and 20 g/l yeast extract. The fermentation was carried out for 4-6 days at 37°C under constant agitation at about 250 rpm in a baffled shake flask. Using a combination of ultrafiltration, gel filtration, and ion exchange chromatography as described below, purified mannanase was obtained. Culture supernatant, 375 ml, enriched for mannanase was concentrated using an Amicon stir-cell (350 ml capacity, PM-10 membrane) to a final volume of 75 ml. 25 ml of this concentrate was applied to a gel filtration column (XK 26/100 packed with Sephacryl S-100 HR, Pharmacia) which had been equilibrated with 10 mM tris-HCI, pH 9.0. The flow rate used was 0.5 ml/min, 15 ml fractions were collected, and UV absorbance was monitored at 280 nm. Mannanase activity in resulting fractions was detected using an RBB-glucomannan substrate assay (see below). Activity eluted in four fractions towards beginning of main absorbance peak. This procedure was repeated two more times; all mannanase active fractions were pooled. 100 ml of pooled material was then applied to an anion exchange chromatography column (FPLC 10/10 column packed with Q-Sepharose, Pharmacia) equilibrated with 10 mM tris-HCl, pH 9.0. Flow rate was 2 ml/min., UV absorbance was monitored at 280 nm as was conductivity, 1.5 ml fractions were collected. After washing the column with 10 ml (2 void volumes), elution was carried out with a 100 ml linear increasing NaCI gradient, from 10 mM tris-HCI, pH 9.0 to 100 mM NaCI in 10 mM tris-HCI, pH 9.0. Mannanase activity in fractions was determined as above, the majority of which was found to elute approximately mid-way through the salt gradient, in four fractions. Degree of purity was determined using silver stained isoelectric focusing (IEF) gels on a PhastSystem (Pharmacia). IEF gels revealed homogeneous mannanase in the first three of the aforementioned four fractions.

### Example 2

### Characterization and Properties of Mannanase from Bacillus amyloliquefaciens

*TECHNIQUES*. Relative mannanase activity was determined using a remazol brilliant blue dyed birchwood glucomannan (RBB-mannan) substrate (Megazyme, Sydney, Australia). Samples, 200 µl, were mixed with 250 µl of substrate solution (2% (w/v) RBB-mannan in 300 mM sodium acetate pH 4.5) and incubated at 40°C for 10 minutes. Undigested mannan was precipitated by the addition of 1 ml 95% ethanol and removed by centrifugation. Released dye remaining in solution was quantified by spectrophotometry (OD₅₉₀) and was proportional to mannanase activity.

Mannanase activity was quantified using a DNS method for the quantification of resulting reducing sugars. Sample, 200 µl, was mixed with 1.8 ml galactoglucomannan substrate (0.5% (w/v) locust bean gum in 50 mM sodium citrate, pH 5.3) and incubated for 10 min. at 50 °C. DNS solution (1% (w/v) dinitrosalicylic acid containing 30% (w/v) sodium-potassium tartrate and 1.6% (w/v) NaOH), 3 ml, was added and solution boiled for 5 minutes. OD at 540 nm was measured and was a function of sugar release/mannanase activity as compared to a standard curve. Units are reported in nkat/ml.

Isoelectric focusing (IEF) and sodium dodecyl sulfate polyacrylamide gel electrophoresis (SDS PAGE) were carried out using a PhastSystem (Pharmacia) as per manufacturer's instructions. Markers used for pl determination were a broad pl kit pH 3.5-9.3 (Pharmacia Biotech). Molecular weight markers used were from Sigma Chemical Co. (St. Louis, MO). Visualization of proteins was by PhastSystem development silver staining, as per instructions.

Protein concentrations were determined using a BCA method (Pierce Co.).

Molecular weight determinations were accomplished by SDS-PAGE and by gel filtration as follows: Using a Pharmacia FPLC system, 1 ml purified mannanase was applied to two gel filtration columns linked in tandem (Pharmacia Superdex G-200 10/30 followed by Pharmacia Superdex G-75 10/30) which had been equilibrated with 100 mM NaCl-50 mM citrate/phosphate buffer, pH 6.0. Flow rate was 0.5 ml/min., UV absorption was monitored at 280 nm, 1 ml fractions were collected. Fractions were assayed for mannanase activity using an RBB-mannan substrate assay. Mannanase activity was found to elute after 52.5 minutes using this system. Pharmacia low molecular weight gel filtration standards (1.25 mg/ml) were applied to the system using the above conditions and elution results were used to create a molecular weight standard curve. Elution of *Bacillus* mannanase corresponded to a molecular weight of 18-22 kilodaltons when compared to the standard curve.

Thermostability and alkaline stability was determined by adjusting described temperature and pH and assaying as above using the RBB-mannan assay.

A gel overlay method for detecting the presence of multiple mannanases and to determine their isoelectric point (pl) was also developed using RBB-mannan substrate. IEF gels, pH 3-9, were overlaid with a melted agarose-substrate suspension (4% (w/v) agarose, 7 mg/ml RBB-mannan, 0.5% (v/v) glycerol in 50 mM sodium acetate, pH 4.5) and incubated at 37°C. After 1 hour xylanase activity was evident as clearing zones. Gels were allowed to dry completely and stored. Mannanase pl was determined by comparison with identically run IEF gels containing silver stained pl standards.

**Table 1**

| Purified Mannanase Characteristics | |
|---|---|
| | *Bacillus amyloliquefaciens* |
| MW (kD, SDS-PAGE) | 33-37 |
| MW (kD, Gel Filtration) | 18-22 |
| Specific Activity (nkat/mg) | approx. 10500 |
| pl | 5.2-5.6 |
| pH Optimum | 4.8-5.2 |
| Temp. half life, 80 °C | approx. 45 sec. |

### Example 3

### Pulp Bleaching with Mannanase Based Composition

The bleaching sequence contained the following stages: enzyme treatment, chelation and two alkaline peroxide stages. The enzyme treatment stage was run under the same conditions as described above, except that 10% consistency and culture supernatants enriched for mannanase (purified to be free of cellulase and most xylanase) were used. The enzyme stage filtrate was acid hydrolyzed and total sugars were detected by HPLC. In the chelation stage, 0.2% EDTA was used at pH 5.5 and 85°C for 30 min at 3% consistency to remove the metals. The peroxide stages were carried out at 10% consistency at 85°C for 4 hours. In the first peroxide stage the H202 concentration was 3.5% and NaOH concentration 2.2%, in the second the concentrations were 1.5 and 0.85% respectively. After the bleaching stages the pulp was acidified, and handshets were prepared for brightness measurement. The brightness was measured according to the ISO 2469 method. Kappa number, which represents pulp lignin concentration was measured by the SCAN-C1:77 method. Table 2 illustrates the results of pulp bleaching using the XQPP Sequence. "xyl + mann 200" indicates Irgazyme 40 treatment (0.3 l/t) plus mannanase dosed at 200 nkat/g pulp. Monomeric sugars have been detected by HPLC after acid hydrolysis of the enzyme filtrate ("X Stage").

**Table 2**

| Enzyme | X-stage total carbohyd. (g/l) | Brightness ISO % | Kappa |
|---|---|---|---|
| Reference (no enzymes) | 0.048 | 80.4 | 5.0 |
| Irgazyme Only 0.3 l/t | 0.954 | 81.1 | 4.4 |
| *Bacillus* xyl + mann 200 | 1.548 | 82.5 | 4.2 |
| *Bacillus*: mann 200 only | 0.394 | 80.7 | 4.9 |

Of course, it should be understood that a wide range of changes and modifications can be made to the preferred embodiments described above. It is, therefore, intended to be understood that it is the following claims, including all equivalents, which define the scope of the invention.

## Claims

1. A mannanase obtained from *Bacillus amyloliquefaciens,* **characterized in that**:
said mannanase has a molecular weight of about 33-37 kD as determined by SDS-PAGE;
said mannanase has a pl of about 5.2-5.6; and
said mannanase has a pH optimum of about 4.8-5.2.

2. The purified mannanase according to claim 1, wherein said mannanase has a half-life of about 45 seconds at a temperature of about 80°C.

3. A method for producing a purified mannanase according to claim 1 comprising:
(a) preparing a fermentation broth of *Bacillus amyloliquefaciens*;
(b) separating the cells, cell fragments and particulate matter from said fermentation broth to purify said mannanase;
(c) optionally concentrating said mannanase to produce a concentrated mannanase solution.

4. The process according to claim 3, wherein said mannanase has a half-life of about 45 seconds at a temperature of about 80°C.

5. A process for bleaching pulp comprising contacting said pulp with a composition comprising a mannanase according to claim 1.

6. The process according to claim 5, wherein said composition further comprises xylanase.

## Patentansprüche

1. Mannase, erhalten aus *Bacillus amyloliquefaciens,* **dadurch gekennzeichnet, dass**:
diese Mannase ein Molekulargewicht von etwa 33 - 37 kD aufweist, wie durch SDS-PAGE bestimmt;
diese Mannase einen pI von etwa 5,2 - 5,6 aufweist; und
diese Mannase ein pH-Optimum von etwa 4,8 - 5,2 aufweist.

2. Gereinigte Mannase nach Anspruch 1, wobei die Mannase eine Halbwertszeit von etwa 45 Sekunden bei einer Temperatur von etwa 80 °C aufweist.

3. Verfahren zur Herstellung einer gereinigten Mannase nach Anspruch 1, umfassend:
a) Herstellung einer Fermentationsbrühe von *Bacillus amyloliquefaciens*;
b) Abtrennen der Zellen, Zellfragmente und des teilchenförmigen Materials von der Fermentationsbrühe, um die Mannase zu reinigen;
c) gegebenenfalls Konzentrieren der Mannase, um eine konzentrierte Mannase-Lösung zu erzeugen.

4. Verfahren nach Anspruch 3, in dem die Mannase eine Halbwertszeit von etwa 45 Sekunden bei einer Temperatur von etwa 80 °C aufweist.

5. Verfahren zum Bleichen von Zellstoff, umfassend das Kontaktieren des Zellstoffs mit einer Zusammensetzung, die eine Mannase nach Anspruch 1 umfasst.

6. Verfahren nach Anspruch 5, in dem die Zusammensetzung weiter Xylanase umfasst.

## Revendications

1. Mannanase obtenue à partir du *Bacillus amlyoliquefaciens*, **caractérisée en ce que** :
ladite mannase a un poids moléculaire d'environ 33 - 37 kD, tel que déterminé par SDS-PAGE ;
ladite mannase a un point isoélectrique (pl) d'environ 5,2 - 5,6 ; et que
ladite mannanase a un pH maximum d'environ 4,8 - 5,2.

2. Ammanase purifiée selon la revendication 1, dans laquelle ladite ammanase a une demi-vie d'environ 45 secondes à une température d'environ 80 °C.

3. Procédé de fabrication d'une ammanase purifiée selon la revendication 1, comprenant :
a) la préparation d'un bouillon de fermentation du *Bacillus amlyoliquefaciens ;*
b )la séparation des cellules, des éléments cellulaires et des matières particulaires du dit bouillon de fermentation afin de purifier la dite mannanase ;
c) la concentration optionnelle de la dite mannanase afin de produire une solution concentrée de mannanase.

4. Procédé selon la revendication 3, dans lequel ladite mannanase a une durée de vie d'environ 45 secondes à une température d'environ 80 °C.

5. Procédé pour le blanchiment de la pâte à papier comprenant le contact de la dite pâte à papier avec une composition comprenant une mannanase selon la revendication 1.

6. Procédé selon la revendication 5, dans lequel ladite composition comprend en outre de la xylanase.
